**Europäisches Patentamt**

**European Patent Office**

**Office européen des brevets**

(11) Veröffentlichungsnummer: **0 121 831**
A2

(12) # EUROPÄISCHE PATENTANMELDUNG

(21) Anmeldenummer: **84103037.2**

(51) Int. Cl.³: **A 61 B 5/00,** G 01 N 21/64

(22) Anmeldetag: **20.03.84**

(30) Priorität: **12.04.83 DE 3313047**

(43) Veröffentlichungstag der Anmeldung: **17.10.84**
**Patentblatt 84/42**

(84) Benannte Vertragsstaaten: **AT CH FR GB IT LI LU NL SE**

(71) Anmelder: **Max-Planck-Gesellschaft zur Förderung der Wissenschaften e.V., Bunsenstrasse 10, D-3400 Göttingen (DE)**

(72) Erfinder: **Lübbers, Dietrich Werner, Prof. Dr., Rheinlanddamm 201a, D-4600 Dortmund 1 (DE)**
Erfinder: **Opitz, Norbert, Dr. Dipl.-Phys., Villigsterstrasse 6, D-5840 Schwerte 5 (DE)**

(74) Vertreter: **Hofmann, Hans Walter, Dr., Hauberisserstrasse 36, D-6200 Wiesbaden (DE)**

(54) **Anordnung zum Messen von diffundierenden Partikeln.**

(57) Um bei einer Anordnung zur Messung von diffundierenden Partikeln, die mindestens eine monochromatische Strahlungsquelle und eine Lichtmesseinrichtung sowie mindestens einen stofflich abgegrenzten, durch die zu messenden Partikel permeierbaren, mit einem optischen Fluoreszenzindikator versehenen Indikatorraum aufweist, dessen Indikator durch die zu messenden Partikel optisch veränderbar ist, die Messung unter dem Augenlid zu ermöglichen, ist ein Trägerkörper vorgesehen, der Vertiefungen in der am Lid anliegenden Fläche aufweist, in denen die Indikatorräume (Optoden) angeordnet sind.

0121831

— 1 —

ANORDNUNG ZUR MESSUNG VON DIFFUNDIERENDEN PARTIKELN.

Die Erfindung betrifft eine Anordnung zur Messung von diffundierenden Partikeln, die mindestens eine monochromatische Strahlungsquelle und eine Lichtmesseinrichtung, sowie mindestens einen stofflich abgegrenzten, durch die zu messenden Partikel permeierbaren, mit einem optischen Fluorszenzindikator versehenen Indikatorraum aufweist, dessen Indikator durch die Partikel optisch veränderbar ist.

Anordnungen der angegebenen Gattung werden verwendet, um diffundierende Partikel, wie beispielsweise den Blutbestandteil Sauerstoff und dessen Partialdruck $pO_2$ oder die Kohlensäure und deren Partialdruck $pCO_2$ invasiv oder nichtinvasiv zu messen. Dazu werden die von den zu messenden Partikeln permeierbaren Indikatorräume, kurz OPTODEN genannt, mit dem Messobjekt in Wirkverbindung gebracht und die Veränderung des Messlichtes durch die Optode gemessen.

Aus der US-PS 3769961 ist es bekannt, mittels Elektroden, die auf einer als Elektrodenträger ausgebildeten Kontaktlinse angeordnet sind, Blutbestandteile, wie beipielsweise Sauerstoff oder Kohlensäure, am Augenlid zu messen.

Auf diese Weise ist eine nichtinvasive Messung dieser BLutbestandteile ermöglicht, weil auf der Lidschleimhaut oder der Conjunctiva diese Blutbestandteile nahzu arterielle Werte aufweisen, oder nur durch einen konstanten Faktor davon verschieden sind.

Elektroden, wie sie im bekannten Stand der Technik zur Messung verwendet sind, weisen, wie alle Elektroden, eine Alterung auf und müssen häufig mit erheblichem Aufwand gewartet werden. Weiterhin müssen die Drahtzuführungen für die Elektroden auf die Kontaktlinse geführt werden, was zur Beeinträchtigung des Patienten führt. Ausserdem ist die Miniaturisierung von Elektroden nur beschränkt möglich. Auch andere Partikelarten, wie beispielsweise Stoffwechselprodukte aus dem Stoffwechsel des Auges oder andere Partikel, wie beispielsweise Kationen oder Anionen liegen an diesem Messort vor. Sie konnten jedoch bisher dort nicht gemessen werden, weil geeignete Sensoren fehlen.

Um diese Nachteile zu vermeiden, ist ein Trägerkörper vorgesehen, der Vertiefungen in der am Lid anliegenden Fläche aufweist, in denen Indikatorräume (Optoden) angeordnet sind.

Auf diese Weise ist es möglich, beipielsweise gleichzeitig mehrere Partikelarten kontaktlos zu messen, indem die auf dem Trägerkörper angeordneten Optoden nach Anheben des Lides unmittelbar oder durch das Augenlid hindurch mit Prüflicht bestrahlt und dann das von den Optoden ausgehende Messlicht und seine Änderung angezeigt werden. Hiermit ist insbesondere auch eine nichtinvasive Methode zur Messung des pH-Wertes geschaffen, weil Protonen ebenfalls mit nahezu arteriellen Werten am Augenlid messbar sind. Durch die robuste Beschaffenheit der Optoden ist die Anordnung unproblematisch in der Handhabung, und die·Verträglichkeit für den Patienten ist so gut, wie die bekannte Verträglichkeit von Kontaktlinsen. Da es darüberhinaus

optischen Indikatoren für eine ganze Reihe von diffundierenden Teilchen gibt, ist der Messbereich gegenüber der Messung mit Elektroden vergrössert. Beispielsweise kann mit Optoden auch die Glucosekonzentration am Lid gemessen werden.

Eine besonders schonende und zugleich intensive Bestrahlung der Optoden wird dadurch erreicht, dass innerhalb des Trägerkörpers Lichtleiter vorgesehen sind, die von einer oder mehreren am Rande des Trägerkörpers angeordneten Ankopplungsflächen zu den Optoden führen.

Ist unter besonderen Messbedingungen die Verwendung von Drahtzuleitungen zumutbar, dann kann eine monochromatische Strahlungsquelle, beispielsweise eine Leuchtdiode und der zugeordnete Strahlungsempfänger im Trägerkörper angeordnet sein, sodass die Bestrahlung der Optoden und die Messung der Strahlungsänderung durch die Optoden unmittelbar erfolgt. Es können auch mehrere solcher Anordnungen verwendet werden.

In der Zeichnung, anhand derer die Erfindung näher erläutert wird, zeigen

Fig.1:   Eine erste Ausführung der Erfindung

Fig.2:   Eine zweite Ausführung der Erfindung

Fig.3:   Eine Abwandlung der Erfindung

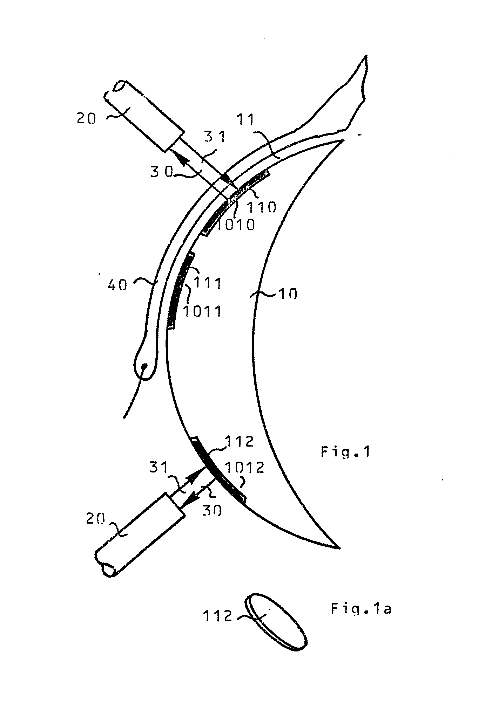

In Fig.1 sind in der am Augenlid anliegenden Fläche 11 eines Trägerkörpers 10 Vertiefungen 1010,1011,.. angeordnet, deren Rückseite reflektierend ausgebildet sein kann, und in die Optoden 110,111.. eingelegt sind. Solche Optoden bestehen beispielsweise aus Kuststoffolien, Fig.1a, in denen Indikatoren angeordnet sind, die bei Bestrahlung mit Licht einer bestimmten, vom Indikator abhängigen Wellenlänge Fluoreszenzlicht erzeugen. Das Fluoreszenzlicht oder

"Messlicht" 30 weist eine andere Farbe auf als das Prüflicht 31. Dieses Messlicht 30, das in Fig.1 ebenso wie das Prüflicht 31 von einem Lichtleiter 20, der mehrer Fasern aufweisen kann, transportiert wird, kann leicht durch bekannte optische Mittel vom Messlicht getrennt und nachgewiesen werden. Die dazu erforderliche Anordnung ist aus der Technik bekannt und nicht eigens gezeichnet.

Der Indikator, der dieses Fluorszenzlicht erzeugt, wird seinerseits von einer zu messenden Partikelart, beispielsweise von Sauerstoff, der von der Innenoberfläche des Augenlids 40 ausgeht und der im Diffusionsgleichgewicht mit dem kapillaren Sauerstoff in der Schleimhaut des Augenlids steht, beeinflusst und ändert seine Fluoreszenzlichtabgabe entsprechend der Konzentration des Sauerstoffes.

Die Messung für $pO_2$ und $pCO_2$ erfolgt durch das Augenlid mittels der Optoden 110, 111.., damit keine Verfälschungen durch die Atmosphäre erfolgen. Das ist deshalb möglich, weil das Fluoreszenzlicht des Indikators gut aus der reflektierten Strahlung ausgefiltert werden kann.

Weiterhin können die Optoden ohne weiteres aus biokompatiblen Stoffen hergestellt werden, sodass weder toxische noch kontaminierende Vorgänge durch die Messung ausgelöst werden. Durch optische Entkopplung der Optoden mittels reflektierender oder absorbierender Schichten kann die bei der Messung mögliche Einstrahlung in die Retina ausgeschaltet werden.

Die derart gemessene Intensität des Fluoreszenzlichtes wird mit einem Korrekturfaktor multipliziert, der durch Eichung ermittelt ist. Die Abweichung der Partialdrucke von den arteriellen Drucken kann durch eine weitere Korrektur eliminiert werden.

Für besondere Messaufgaben kann eine Optode 112 auch im offenen Lidspalt angeordnet sein. Weiterhin können mehrere Optoden, insbesondere zur simultanen Messung mehrere Partikelarten oder physikalischer Parameter, wie beispielsweise der Sauerstoffkonzentration, des $CO_2$-Partialdruckes, des pH-Wertes, der Glucosekonzentration, der Temperatur und dergl, auf der Vorrichtung angeordnet sein.

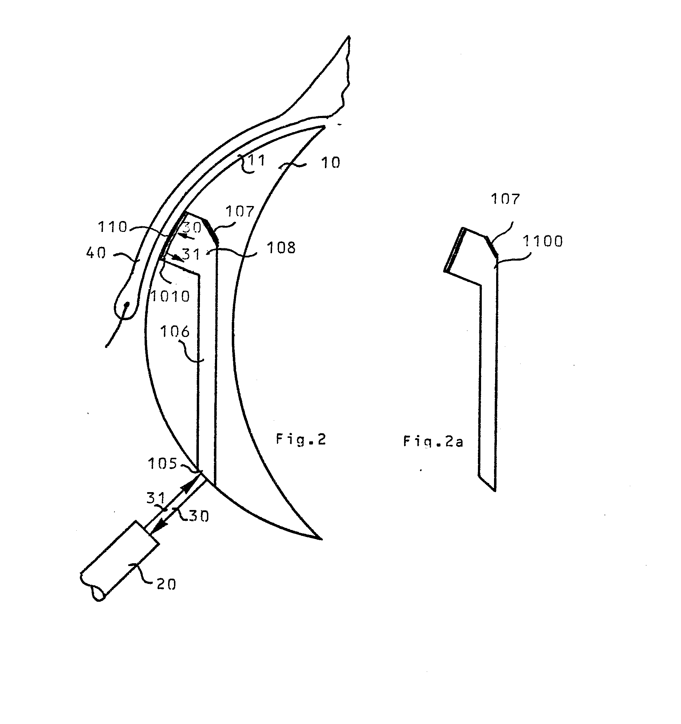

Die Anordnung nach Fig.2 wird dann verwendet, wenn der $pO_2$-Wert, der pH-Wert oder der $pCO_2$-Wert bei geöffnetem Lid gemessen werden und dabei die Beleuchtung nicht durch das Augenlid erfolgen soll.

Dazu ist die Vertiefung 1010 soweit am Rande des Trägerkörpers 10 angeordnet, dass das Lid 40 diese Optode permanent bedeckt. Der das Prüflicht 31 und das Messlicht 30 transportierende Lichtleiter 20 bestrahlt eine Kopplungsfläche 105, die die Endfläche eines Lichtleiters 106 ist, der die Strahlung auf die Optode 110 leitet. Dabei verläuft das Licht beispielsweise über einen Spiegel 107 des Optodenhalters 108

Das sodann von der Optode ausgehende Messlicht läuft auf dem gleichen Wege zurück.

Die Anordnung 1100 in Fig.2a, die eine einstückige Ausführung der Einzelteile: Kopplungsfläche (105), Lichtleiter (106), Spiegel (107), Optodenhalter (108) ist, kann beispielsweise für sich hergestellt und in einen mit den entsprechenden Kanälen versehenen Trägerkörper 10 eingesetzt sein.

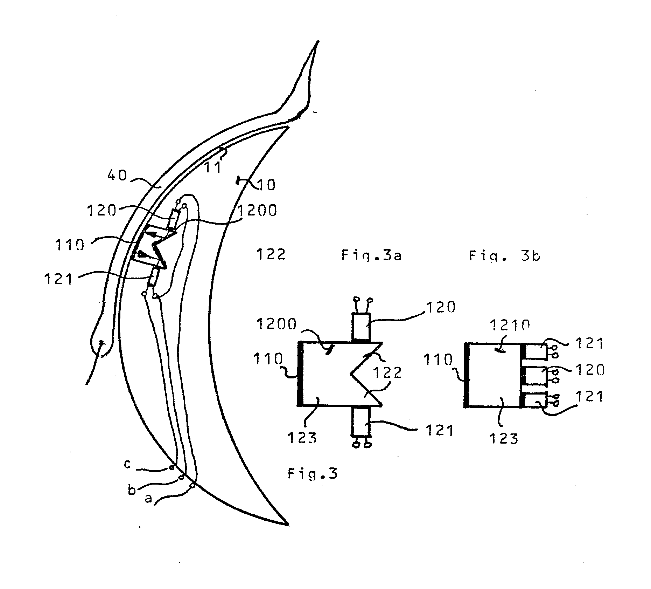

In Fig.3 ist eine Anordnung dargestellt, bei der Zuleitungen a,b,c auf den Trägerkörper zugelassen werden. Dadurch ist es möglich, farbig emittierende Leuchtdioden 120 und Fotoempfänger 121 im Trägerkörper anzuordnen, die über

Zuleitungen a,b gespeist und deren Signale über die Leitungen b,c abgenommen werden.

Dabei können, je nach verfügbarem Raum, Anordnungen nach Fig.3a oder Fig.3b verwendet werden.

Auch in diesem Fall bilden die Anordnungen 1200,1210 aus Leuchtdiode 120, Spiegel 122, Fotoempfänger 121 und Optodenhalter 123 Baueinheiten, die in den Trägerkörper 10 eingelassen sind.

- 7 -

PATENTANSPRÜCHE

1. Anordnung zur Messung von diffundierenden Partikeln, die mindestens eine monochromatische Strahlungsquelle und eine Lichtmesseinrichtung, sowie mindestens einen stofflich abgegrenzten, durch die zu messenden Partikel permeierbaren, mit einem optischen Fluorszenzindikator versehenen Indikatorraum aufweist, dessen Indikator durch die Partikel optisch veränderbar ist, dadurch gekennzeichnet dass ein Trägerkörper (10) vorgesehen ist, der Vertiefungen (1010,1011,..) in der am Lid (40) anliegenden Fläche (11) aufweist, in denen Indikatorräume (Optoden) (110,111,..) angeordnet sind.

2. Anordnung nach Anspruch 1, dadurch gekennzeichnet, dass mehrere Indikatorräume mit Indikatoren zur simultanen Messung mehrerer physikalischer Parameter und/oder mehrer Partikelarten vorgesehen sind.

3. Anordnung nach einem der Ansprüche 1 oder 2, dadurch gekennzeichnet, dass innerhalb des Trägerkörpers (10) Lichtleiter (106,..) vorgesehen sind, die das Licht von einer oder mehreren am Rande des Trägerkörpers (10)

angeordneten Ankopplungsflächen (105) zu den Optoden (110,..) führen.

4. Anordnung nach einem der Ansprüche 1 bis 3, dadurch gekennzeichnet, dass die Messung durch das Augenlid erfolgt.

5. Anordnung nach Anspruch 1, dadurch gekennzeichnet, dass mindestens eine monochromatische Strahlungsquelle (120) und die zugeordneten Strahlungsempfänger (121) im Trägerkörper (10) angeordnet sind und die Bestrahlung der Optoden (110,111,112..) und die Messung der Strahlungsänderung unmittelbar im Trägerkörper (10) erfolgt.

6. Anordnung nach einem der Ansprüche 1 bis 5, dadurch gekennzeichnet, dass Lichtleiteinrichtungen (1100,1200,1210) innerhalb des Trägerkörpers (10) vorgesehen sind.

7. Anordnung nach einem der Ansprüche 1 bis 6, dadurch gekennzeichnet, dass optische Entkopplungen durch reflektierende oder absorbierende Schichten nach der Retina hin erfolgen.

8. Anordnung nach einem der Ansprüche 1 bis 7, dadurch gekennzeichnet, dass eine Lichtmesseinrichtung (1200,1210) innerhalb des Trägerkörpers (10) vorgesehen sind.

9. Anordnung nach Anpruch 6 oder 8, dadurch gekennzeichnet, dass die Lichtleiteinrichtungen (1100) oder auch die Lichtmesseinrichtungen (1200,1210) einstückig ausgebildet und in eine Ausnehmung des Trägerkörpers (10) einsetzbar ist.

20    11
31
30    110
1010

40    111
1011    10

Fig.1

112
31    1012
20    30

112    Fig.1a

Fig.2

Fig.2a

40

11

10

120

1200

110

121

122

Fig.3a

Fig. 3b

1200

120

110

110

122

121

1210

121

120

121

123

123

Fig.3

c

b

a

123